# EUROPEAN PATENT SPECIFICATION

(11) **EP 1 988 968 B1**
(45) Date of publication and mention of the grant of the patent: **14.09.2011**
(21) Application number: 07757341.8
(22) Date of filing: 22.02.2007
(51) Int. Cl.: A61N 5/06, A61N 5/067

(54) **LASER HAIR AND SCALP TREATMENT DEVICE**
LASERBEHANDLUNGSGERÄT FÜR HAAR UND KOPFHAUT
DISPOSITIF DE TRAITEMENT PAR LASER DES CHEVEUX ET DU CUIR CHEVELU

(30) Priority: 22.02.2006 US 776907 P; 07.06.2006 US 804167 P
(43) Date of publication of application: 12.11.2008
(73) Proprietor: Spencer Forrest, Inc., Westport, CT 06880 (US)
(72) Inventor: MULHAUSER, Paul, New York, NY 10003 (US); TREACY, Lyndon, T., Long Island City, NY 11109 (US); KIRK, Karl, D., New York, NY 10014 (US); KRESS, Mark, Westport, CT 06880 (US)
(74) Representative: Fiener, Josef
(86) International application number: PCT/US2007/062593
(87) International publication number: WO 2007/101067

(56) References cited:
- US-A1- 2002 138 929
- US-A1- 2002 188 334
- US-A1- 2004 006 332
- US-A1- 2004 243 145
- US-B1- 6 802 853

## Description

### Background of the Invention

### Area of the Art

The current invention is in the area of topical scalp treatment with electromagnetic radiation and more particularly to a hand held device that delivers laser light to the scalp in an amount and distribution to stimulate hair growth.

### Description of the Background Art

Scalp hair in humans serves a number of important biological functions probably the most important being protection of the skull from blows and from sunburn. Yet humans of both sexes have long been enthralled by scalp hair to an extent far beyond its apparent biological importance. It is generally considered an important asset in the appearance of both males and females. Vast amounts of money are spent on cleaning, coloring and styling hair. When a person loses hair either due to disease or natural biological processes like male pattern baldness, that person will often go to great lengths to overcome the loss. Folk medicine is filled with numerous baldness cures based on herbs and various more or less bizarre procedures. Wigs and hairpieces have been used for thousands of years. A wide variety of cosmetic procedures are available to minimize the appearance of hair loss. Yet only in relatively recent time have methods been developed to actually return growing hair to the scalp.

Probably the oldest of these methods is surgical hair replacement. This technique takes living hair follicles from a region that has hair and transplants them into a region that lacks hair. Surgical methods are expensive and painful and may involve side effects such as infection. In addition, many persons lack adequate areas of normal hair growth so as to provide sufficient follicles to repopulate the scalp. As a result pharmaceutical means to promote hair growth have been developed. Probably the first was the topical application of minoxidil. While pharmaceutical approaches show some effectiveness, they are generally unable to reverse a case of profound baldness. In addition, both topical and systemic pharmaceutical agents can provoke a number of different side effects.

More recently it has been discovered that carefully targeted light treatments may positively effect hair growth. Generally the most effective treatments have involved application of laser light at red and far red wavelengths. The precise reason for efficacy is unknown and is it not certain that laser radiation is required. Most likely the laser sources are simply more efficient at delivering effective doses of light. The levels of effective laser radiation are relatively low, but it has been found that it is important to deliver sufficient radiation evenly to the scalp. Because lasers were originally rather bulky optical devices early units intended to accomplish such treatments incorporated cumbersome helmets and tended to be large units resembling X-ray machines or other instruments common in a medical setting. With the advent of solid state (diode) lasers it became possible to reduce the treatment device into a more or less hand held unit. An example of an early version of such a device can be found in U.S. Patent No. 6,497,719 to Pearl et al. In that disclosure the laser source is embedded within a handheld comb or brush unit designed so that the tines or bristles part the hair to allow the laser light to bathe the surface of the scalp.

A drawback of devices that mimic combs or brushes has been an inability to deliver a consistent and reproducible level of laser radiation to the surface of the scalp. One approach is to have one or more laser sources (such as laser diodes) within the body of the device and to deliver the laser light through an optical system usually consisting of lenses and/or reflectors. The laser radiation can be blocked by hairs on the scalp although teeth (as in a comb) or bristles (as in a brush) can be provided to selectively reveal portions of the scalp to the laser beam. Nevertheless, much laser radiation may still be blocked by hair. German Patent No. 9102407 to Mink discloses an improvement over typical teeth or bristles by coupling each laser diode to a light pipe which also acted as a tooth in a comb-like structure. Since the end of a comb tooth directly contacts the scalp, this device was able to deliver radiation directly to the surface of the scalp. However, it is difficult to move such a comb-like device evenly over the scalp surface to ensure even treatment.
Document US 2004/0006332 discloses a laser handheld device according to the preamble of claim 1.

The current invention is a handheld device that utilizes branched light pipes so that each laser diode is able directly to irradiate a plural of points on the scalp. The points can be distributed so that a relatively large area of the scalp can be evenly irradiated at one time.

### Summary of the Invention

This object is achieved with a handheld device according to claim 1.

An improved handheld device is designed for providing laser light treatment to the scalp or other areas of the body benefiting from such treatment. It has been discovered that red and infra red laser light treatments penetrate the skin surface and stimulate growth of hair. Earlier devices intended to take advantage of this discovery suffered from either bulk and expense or from an inability to evenly deliver light treatment in spite of the presence of hair which absorbs or deflects the light. The present device combines a plurality of miniature laser light sources, such as laser diodes, with branched light pipes to distribute and deliver the laser light to the scalp surface. Each diode delivers light to multiple points on the scalp surface by means of the branched light pipe. Each individual light pipe acts like the tooth of a comb and moves interfering hair aside so that the end of the light pipe physically contacts the skin surface and directly conducts the laser light into the skin.

Not only do the branched light pipes allow each individual laser source to directly deliver light to multiple points on the scalp, use of a trifurcated light pipe results in a stable tripod design for each laser source. Just as a stool with three legs can sit evenly on an irregular surface such a tripod allows all the light pipes from a single source to make contact with the scalp in spite of scallop curvature and individual irregularities. By independently mounting each laser source on a flexible member the axis of each laser and its associated branched light pipe can orient independently. This further enhances the ability of the light pipe arrangement to make excellent contact with the scalp surface and deliver and even and repeatable light treatment.

A preferred design combines at least for or five laser sources equipped with trifurcated light pipes into a round disc-shaped device that is easily held in the hand. Such a device provides even laser illumination over a circular region with a diameter of about 3 inches (about 7.6 cm). The device includes flexible mounting of the laser sources to allow independent orientation and a protective membrane that prevents dirt or debris from penetrating. The device is equipped with a timer so that after the unit is energized and placed on the scalp, a signal is given at the appropriate interval so that the device can be moved to achieve complete and even coverage of the scalp. A storage base is provided so that the ends of the light pipes are protected when the device is not in use. In addition the storage base acts as a charging station to recharge batteries in the handheld unit. The use of rechargeable batteries ensures that the device is completely safe electrically since only low battery voltage is present when the device is in contact with a user's scalp.

### Description of the Figures

FIGURE 1 shows a perspective view of the branched light pipe in relation to a laser diode;

FIGURE 2 is shows a perspective view of the branched light pipe engaged with the laser diode;

FIGURE 3 shows one design for a molded prism used to couple the laser diode to the branched light pipe;

FIGURE 4 shows an alternative design for the molded prism used to couple the laser diode to the branched light pipe;

FIGURE 5 shows a diagrammatic cross-section of a branched light pipe illustrating the branching of the laser light beam;

FIGURE 6 shows a diagram of the conduction of laser beams within a trifurcated light pipe;

FIGURE 7 illustrates the coupling of trifurcated light pipes to a flexible membrane;

FIGURE 8 illustrates the branched light pipes making contact with the curved surface of a user's scalp.

FIGURE 9 illustrates the use of a second flexible membrane to prevent the entry of dirt into the device;

FIGURE 10A and 10B show possible distribution patterns of trifurcated light pipes;

FIGURE 11 is a perspective view of a device incorporating branched light pipes;

FIGURE 12 is a side view of the device of Fig. 11;

FIGURE 13 shows a perspective view of the device of Fig. 11 showing the light pipes on the device's lower surface;

FIGURE 14 is a perspective view of the device of Fig. 11 along with a charging station.

### Detailed Description of the Invention

### Description of the Invention

The current invention avoids the problems of the prior art by providing a plurality of light pipes distributed so as to evenly deliver laser radiation to the surface of the scalp. To make efficient use of each laser diode, multiple light pipes are arranged to distribute light from each single laser diode. The larger the number of light pipes per laser diode, the lower the amount of radiation coming from any one light pipe due both to the splitting of the primary beam and also to light loss within the light pipe. An optimal number of light pipes for current laser diodes is probably three. This number could be reduced to two or increased to larger numbers particularly with the use of more powerful diodes. However, there are particular advantages to provide three light pipes for each laser diode. Each light pipe acts as a tine or tooth in a comb to bring laser radiation to the scalp free of obstruction by hair, etc., and a tripod configuration allows each light pipe assembly to evenly contact the rounded surface of the scalp.

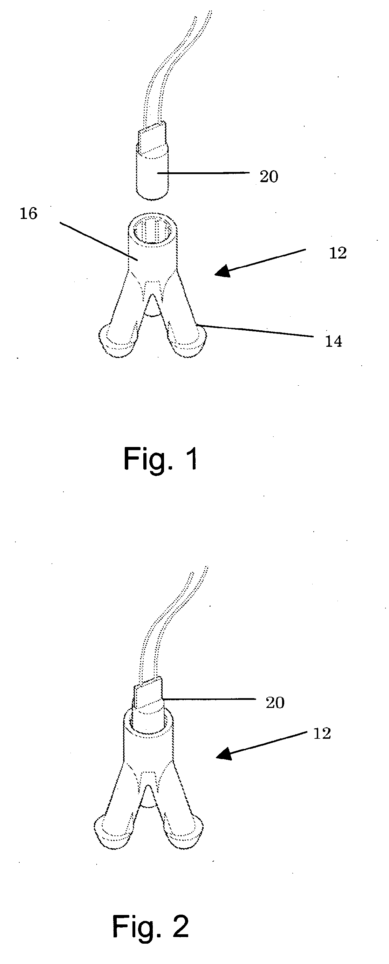

Figs 1 and 2 illustrate the concept of connecting a laser (diode) to a light pipe array with multiple (three according to the invention) branches. Fig. 1 shows the cylindrical laser diode 20 in relation to a branched light pipe tripod 12. The light pipe 12 has an upper region 16 dimensioned to be efficiently optically coupled to the laser diode 20. As is known to one of skill in the art, optical coupling can be achieved by simply bringing the laser diode 20 into optical alignment with the light pipe upper region 16. However, improved results may be obtained by using an optically transmissive agent (such an oil or transparent cement having the proper index of refraction). It will be appreciated that there may be appreciable loss of light within the light pipe 12; however, it is relatively simple to select a laser diode 20 of sufficient power to provide the optimum and safe level of laser radiation at the end of each terminal light pipe 14. In the figure the lower region of the branched light pipe 12 is divided into three separate terminal light pipes or tines 14 equally spaced apart (i.e., at approximately 120 degree intervals) although other numbers of tines could be used and the separation does not necessarily have to be equal, a trifurcation provides certain mechanical advantages-namely a tripod configuration has the advantage of allowing each of the three terminal light pipes 14 of each branched light pipe 12 to contact the surface of the scalp regardless of the scalp's curvature. The branched light pipe 12 is molded or formed from an optically clear plastic such as polystyrene or an acrylic plastic such as polymethacrylate or even from glass. Fig. 2 shows that a single laser beam 18 from the laser diode 20 is divided into three equivalent beams 22 each exiting from one of the light pipes 14.

The figures show the direct coupling of a laser diode to the top surface of the light pipe assembly. One potential problem with such a connection is that there may be suboptimal levels of laser radiation at the end of the times due to inefficient coupling of the incoming laser beam to the upper region 16 of the light pipe 12. This does not seem to be eliminated by optical cements or other approaches to enhancing optical coupling. It appears that the light entering the light pipe may not be at the optimum optical angle or direction for most efficient conduction through the light pipes. As a result light is lost through the walls of the light pipes rather than exiting through the ends of the tines 14 as is desired. One solution to this problem is to mold a "prism" into the top surface of the light pipe. In this case "prism" does not indicate a complete optical prism in which flat surfaces are used to create internal reflections. In the current use "prism" refers to a configuration of flat surfaces which are used to more accurately refract the light from the laser into the individual branches 14 of a branched light pipe 12. Each face or facet 24 of the prism is at approximate' right angles to the long axis of its respective light pipe branch so as to optimally conduct light into that branch.

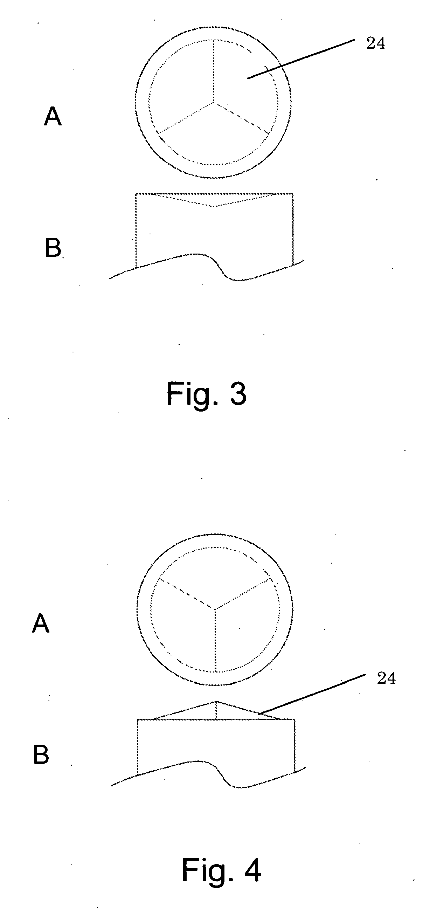

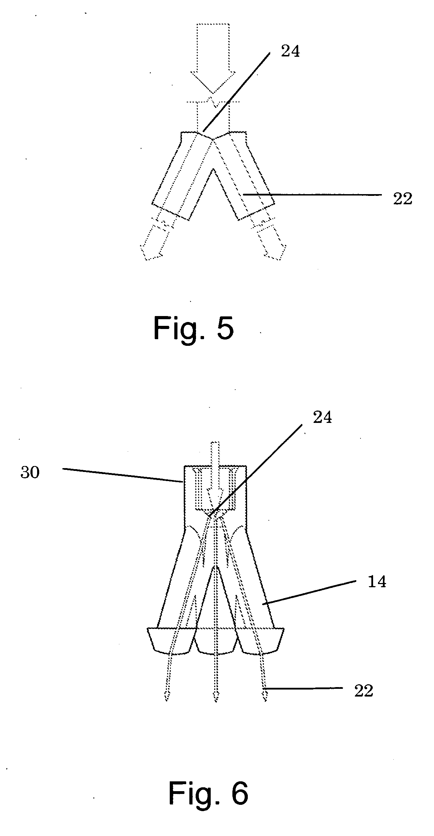

The facets 24 that make up the prism can be either embossed (Fig. 3, top view A, side, view B) (i.e., protruding) or debossed (Fig. 4, top view A, side view B) (i.e., indented). The general configuration is that the number of prism faces 24 is equal to the number of tines or branches 14 in the branched light pipe 12. Essentially, each face 24 is located and angled to maximize the amount of light entering each tine 14 at an angle to ensure optimal transmission as is diagrammed in Fig. 5. Note that in the figure the facets 24 are normal to the axis of each laser beam 22.

As shown in Fig. 6 a recessed collar 30 surrounds the laser so that the light emitting surface of the laser is in close proximity to the faces 24 of the debossed prism. This causes the laser beam to become divided into three direct beams 22-each optimally oriented for transmission by one of the tines 14. To further enhance light transmission the ends 34 of the tines 14 can advantageously be rounded or lens-shaped. The single laser beam from the laser diode 20 is directed towards the center of the prism. Portions of the beam are refracted by each facet thereby directing each portion of the split beam into a separate terminal light pipe or tine 14.

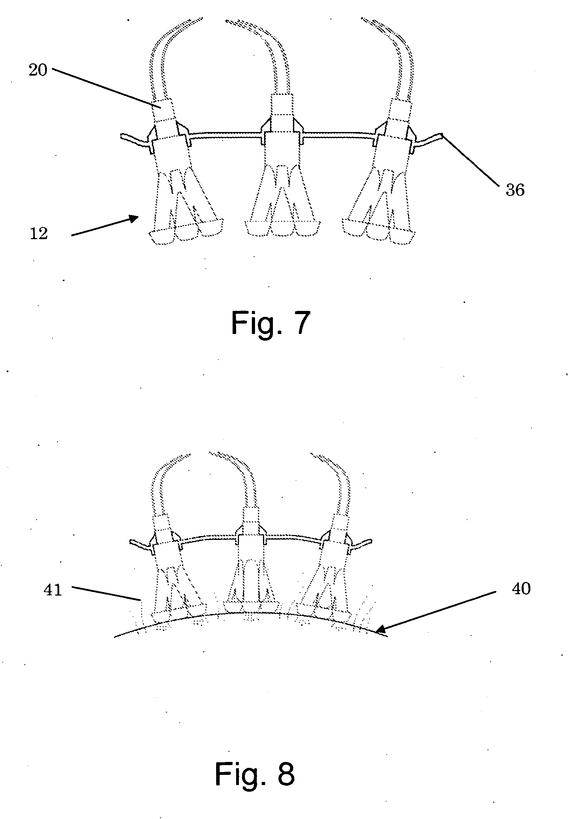

Although it is possible to attach the lasers to a flat (or more likely curved) rigid member, a further improvement in coupling the laser radiation to the scalp can be achieved by attaching the lasers 20 to a flexible member 36 (such as a sheet or band of neoprene, polyurethane or some other resilient elastomer-alternately a separate flexible member attached each laser diode 20 to the device). As shown in Fig. 7 this allows the laser diodes 20 and their attached branched light pipes 12 to reorient in response to changes in the geometry of any surface that is contacted. That is, each tripod will naturally make contact with a flat or curved surface. However, if the surface has irregularities such as ridges, adjacent tripods may not all be able to accommodate themselves to the surface if the axis of the laser is fixed. If, however, the laser is attached to a flexible member 36, each laser 20 can individually reorient so that its light pipes make full contact with the surface. In the Fig. 8 the curved surface 40 represents the surface of a user's scalp (with hair 41) receiving light radiation from the device. This same effect can also be achieved by molding the branched light pipes14 out of an elastomer so that the light pipes themselves flex to ensure contact with the surface of the scalp.

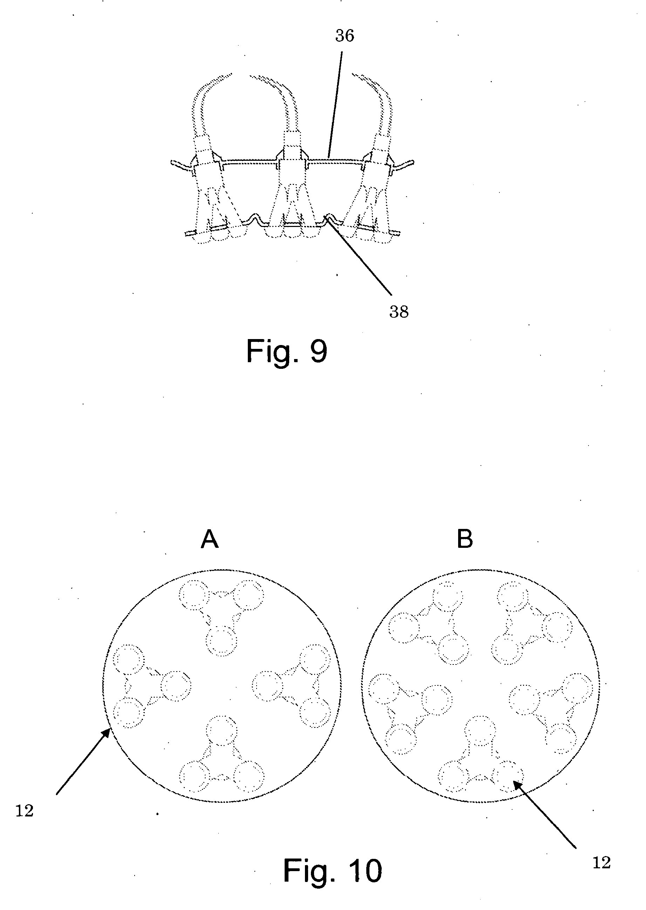

As shown in Fig. 9 an additional improvement can be achieved by providing a sealing membrane 38. The sealing membrane 38 is quite close to the ends of the light pipes 14 which come into contact with the scalp. The tips of each light pipe 14 pass snugly or sealingly through openings in the sealing membrane 38. This arrangement makes the device easier to clean because hair and other debris cannot become tangled around the bodies of the light pipes. Only relatively short blunt ends of the pipes protrude through the sealing membrane so that the whole arrangement can be easily cleaned with a sponge or a soft cloth. Alternatively, sealing membrane can be represented by a rigid layer with the light pipes passing slidingly through openings in the layer. In such an embodiment there is sufficient free space in the openings to allow the light pipes to move relative to the rigid layer, however, the openings are sufficiently small so as to restrict the access of dirt and debris. Other variations such as an o-ring or similar elastic material surrounds each light pipe passing through an opening so as to further restrict entry of contaminants will be apparent to one of skill in the art.

Fig. 10 shows a view of the branched light pipes 12 as seen from the scalp surface. The figure shows that a small number (four in Fig. 10A and five in Fig. 10B) of laser diodes can be spaced apart in a circular pattern to give even coverage of a portion of the scalp. If the circle is sufficiently large and if the density of the diodes is sufficiently high, essentially all regions on the scalp can be simultaneously irradiated. In most practical applications, the circle will be smaller (about three inches or about 7.6 cm in diameter) and/or the density will be lower than that necessary to achieve simultaneous irradiation of the entire scalp surface. Therefore, the device needs to be moved about on the scalp surface to achieve even irradiation. Because the adaptive flexible membrane 36 allows the light pipe ends to automatically accommodate to the scalp surface, the device is easy to maintain in one position on the scalp and easy to move to a second position on the scalp. Such ease of repositioning makes it relatively simple to attain even treatment of the entire scalp.

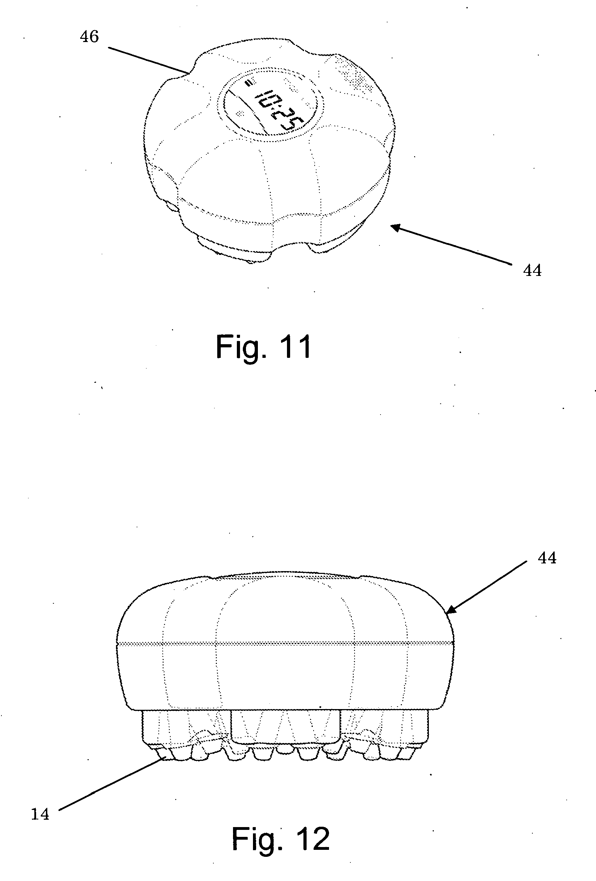

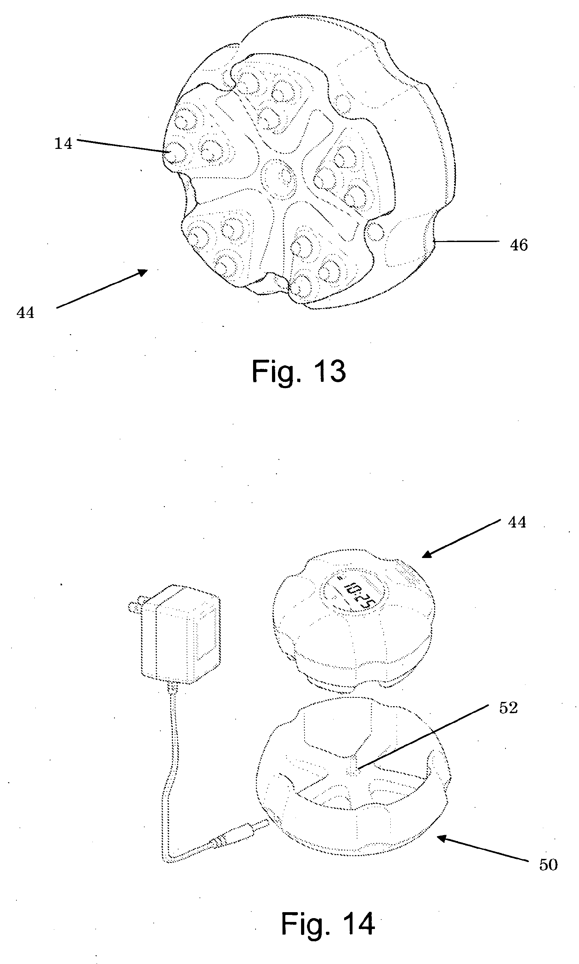

The goal of achieving even treatment is favored by relatively low profile circular device that is shaped so as to be easily grasped. Fig. 11 shows such a domed circular device 44 with circumferential indentations 46 at approximately 72 degree intervals (five indentations) to facilitate grasping the unit. The unit is a convenient size for grasping, preferably between about three and about five inches in diameter. This device is equipped with a lower flexible membrane through which the light pipes 14 protrude. The laser diodes are located in the region of the device body between the indentations. Although relatively flat in profile, there is sufficient room within the body of the device for the necessary electronics. Fig. 12 shows a drawing of the device 44 in side view illustrating the protrusion of the light pipes 14. Fig. 13 shows the device 44 from the lower surface allowing one to see that one branched light pipe (three protruding tines 14) is present in each of the five segments defined by the indentations 46. The unit is preferably powered by rechargeable batteries. Fig. 14 shows a storage stand 50 for the device 44 which serves two purposes: 1) it protects the light pipes 14 when the unit is not in use; and 2) it provides and electrical connection 52 so that the unit automatically recharges while sitting in the storage stand 50.

The unit 44 is used by removing it from the storage stand 50 and activating the laser diodes. The unit is then placed on the scalp and moved about at intervals to ensure even laser light exposure to the region to be treated. The device may be equipped with an integral timer and display to indicate duration of treatment. Periodic sounds (beeps, etc.), lights, tactile ticks or other clues may be used to indicate when the device 44 should be moved. Preferably at the end of the treatment cycle the device automatically turns off to avoid leaving it on by accident.

The following claims are thus to be understood to include what is specifically illustrated and described above, what is conceptually equivalent, what can be obviously substituted and also what essentially incorporates the essential idea of the invention. Those skilled in the art will appreciate that various adaptations and modifications of the just-described preferred embodiment can be configured without departing from the scope of the invention. The illustrated embodiment has been set forth only for the purposes of example and that should not be taken as limiting the invention. Therefore, it is to be understood that, within the scope of the appended claims, the invention may be practiced other than as specifically described herein.

## Claims

1. A handheld device for applying laser light to the scalp comprising:
a housing containing an electronic circuit and a power source for the electronic circuit;
a plurality of miniature electronically powered laser light sources operatively connected to the electronic circuit;
a plurality of branched light pipes optically coupled to each of said laser light sources, each branch of each branched light pipe positioned so as to contact the surface of the scalp and to conduct laser light directly to the surface of the scalp whereby laser light is applied to the scalp **characterised in that** each branched light pipe comprises the branches.

2. The device according to Claim 1, wherein the number of miniature electronically powered laser light sources is selected from the group consisting of four and five.

3. The device according to Claim 1, wherein each branched light pipe comprises an integral prism to refract laser light into each of the branches.

4. The device according to Claim 3, wherein each integral prism comprises a structure selected from the group consisting of embossed faces and debossed faces.

5. The device according to Claim 1, wherein distal ends of the branches of each branched light pipe are rounded.

6. The device according to Claim further comprising a flexible member to which is mechanically coupled the miniature electronically powered laser light sources so that each miniature electronically powered laser light source with its optically coupled branched light pipe can be independently reoriented.

7. The device according to Claim 1 further comprising a sealing membrane disposed in proximity to the distal end of each branch of the branched light pipe to prevent contamination of the proximal ends of the branched light pipes

## Patentansprüche

1. Handgeführte Vorrichtung für das Anwenden von Laserlicht auf der Kopfhaut, umfassend:
ein Gehäuse, das einen elektronischen Stromkreis und eine Stromquelle für den elektronischen Stromkreis enthält;
eine Mehrzahl von elektronisch betriebenen Minlatur-Laserlichtquellen, die wirksam mit dem elektronischen Stromkreis verbunden sind;
eine Mehrzahl von verzweigten Lichtleitern, die optisch mit jeder der Laserlichtquellen verbunden sind, wobei jede Verzweigung des verzweigten Lichtleiters jeweils so positioniert ist, um die Oberfläche der Kopfhaut zu berühren und um das Laserlicht direkt zur Oberfläche der Kopfhaut zu leiten, wodurch Laserlicht auf die Kopfhaut appliziert wird,
**dadurch gekennzeichnet, dass**
jeder verzwelgte Lichtleiter diese Verzweigungen umfasst.

2. Vorrichtung nach Anspruch 1, wobei die Anzahl der elektronisch betriebenen Miniatur-Laserlichtquellen von der Gruppe ausgewählt wird, die aus vier und fünf besteht.

3. Vorrichtung nach Anspruch 1, wobei jeder verzweigte Lichtleiter ein integrales Prisma umfasst, um Laserlicht in jede der Verzweigungen zu lenken.

4. Vorrichtung nach Anspruch 3, wobei jedes integrale Prisma eine Struktur umfasst, die von der Gruppe ausgewählt wird, die aus geprägten Flächen und gestanzten Flächen besteht.

5. Vorrichtung nach Anspruch 1, wobei distale Enden der Verzweigungen jedes verzweigten Lichtleiters abgerundet sind.

6. Vorrichtung nach Anspruch 1, weiterhin umfassend ein flexibles Element, das mechanisch mit den elektronisch betriebenen Miniatur-Laserlichtquellen verbunden ist, um jede elektronisch betriebene Miniatur-Laserlichtquelle mit seiner optisch verbundenen, verzweigten Lichtquelle unabhängig auszurichten.

7. Vorrichtung nach Anspruch 1, weiterhin umfassend eine Dichtungsmembran, die in der Nähe zum distalen Ende jeder Verzweigung des verzweigten Lichtleiters angeordnet ist, um eine Verschmutzung der proximalen Enden der verzweigten Lichtleiter zu verhindern.

## Revendications

1. Dispositif portatif pour appliquer une lumière laser sur le cuir chevelu, comprenant :
un boîtier contenant un circuit électronique et une source de puissance pour le circuit électronique ;
une pluralité de sources de lumière laser miniatures actionnées électroniquement, raccordées de manière opérationnelle au circuit électronique ;
une pluralité de conduits de lumière ramifiés optiquement couplés à chacune desdites sources de lumière laser, chaque ramification de chaque conduit de lumière ramifié étant positionnée afin d'être en contact avec la surface du cuir chevelu et conduire la lumière laser directement à la surface du cuir chevelu, moyennant quoi la lumière laser est appliquée sur le cuir chevelu, **caractérisé en ce que** chaque conduit de lumière ramifié comprend trois ramifications.

2. Dispositif selon la revendication 1, dans lequel le nombre de sources de lumière laser miniatures actionnées électroniquement est choisi dans le groupe comprenant quatre et cinq.

3. Dispositif selon la revendication 1, dans lequel chaque conduit de lumière ramifié comprend un prisme solidaire pour réfracter la lumière laser dans chacune des ramifications.

4. Dispositif selon la revendication 3, dans lequel chaque prisme solidaire comprend une structure choisie dans le groupe comprenant les faces en relief et des faces en creux.

5. Dispositif selon la revendication 1, dans lequel les extrémités distales des ramifications de chaque conduit de lumière ramifié sont arrondies.

6. Dispositif selon la revendication 1, comprenant en outre un élément flexible auquel sont mécaniquement couplées les sources de lumière laser miniatures actionnées électroniquement, de sorte que chaque source de lumière laser miniature actionnée électroniquement avec son conduit de lumière ramifié optiquement couplé, peut être indépendamment réorientée.

7. Dispositif selon la revendication 1, comprenant en outre une membrane d'étanchéité disposée à proximité de l'extrémité distale de chaque ramification du conduit de lumière ramifié pour empêcher la contamination des extrémités proximales des conduits de lumière ramifiés.
